(19) **Europäisches Patentamt / European Patent Office / Office européen des brevets**

(11) **EP 2 061 369 B1**

(12) **EUROPÄISCHE PATENTSCHRIFT**

(45) Veröffentlichungstag und Bekanntmachung des Hinweises auf die Patenterteilung:
**07.12.2011 Patentblatt 2011/49**

(21) Anmeldenummer: **07815184.2**

(22) Anmeldetag: **15.11.2007**

(51) Int Cl.:
**A61B 3/032** (2006.01)

(86) Internationale Anmeldenummer:
**PCT/AT2007/000518**

(87) Internationale Veröffentlichungsnummer:
**WO 2008/064379 (05.06.2008 Gazette 2008/23)**

(54) **VORRICHTUNG ZUM ERFASSEN DER LESE-SEHSCHÄRFE**

DEVICE FOR DETECTING READING ACUITY

DISPOSITIF POUR DÉCELER L'ACUITÉ VISUELLE DE LECTURE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **27.11.2006 US 861174 P**
**27.11.2006 AT 8322006 U**

(43) Veröffentlichungstag der Anmeldung:
**27.05.2009 Patentblatt 2009/22**

(73) Patentinhaber:
- **Dexl, Alois K.**
  **5020 Salzburg (AT)**
- **Grabner, Günther**
  **1010 Wien (AT)**
- **Schlögel, Horst**
  **8010 Graz (AT)**
- **Wolfbauer, Michael**
  **8046 Stattegg (AT)**

(72) Erfinder:
- **Dexl, Alois K.**
  **5020 Salzburg (AT)**
- **Grabner, Günther**
  **1010 Wien (AT)**
- **Schlögel, Horst**
  **8010 Graz (AT)**
- **Wolfbauer, Michael**
  **8046 Stattegg (AT)**

(74) Vertreter: **Patentanwaltskanzlei Matschnig & Forsthuber OG**
**Siebensterngasse 54**
**1071 Wien (AT)**

(56) Entgegenhaltungen:
**US-A- 5 568 209     US-A1- 2003 218 721**
**US-A1- 2006 078 858     US-B1- 6 310 644**
**US-B1- 6 310 644     US-B1- 6 543 898**

- **HITZENBERGER C K: "OPTICAL MEASUREMENT OF THE AXIAL EYE LENGTH BY LASER DOPPLER INTERFEROMETRY", INVESTIGATIVE OPHTHALMOLOGY & VISUAL SCIENCE, ASSOCIATION FOR RESEARCH IN VISION AND OPHTHALMOLOGY, US, vol. 32, no. 3, 1 March 1991 (1991-03-01), pages 616-624, XP000923153, ISSN: 0146-0404**
- **HITZENBERGER C K: "OPTICAL MEASUREMENT OF THE AXIAL EYE LENGTH BY LASER DOPPLER INTERFEROMETRY", INVESTIGATIVE OPHTHALMOLOGY & VISUAL SCIENCE, ASSOCIATION FOR RESEARCH IN VISION AND OPHTHALMOLOGY, US, vol. 32, no. 3, 1 March 1991 (1991-03-01), pages 616-624, XP000923153, ISSN: 0146-0404**

EP 2 061 369 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft eine Vorrichtung zum Erfassen der Sehschärfe (Visus) gemäß dem Oberbegriff des Anspruchs 1, speziell beim Lesen von Texten (Nah- bzw. Lesevisus, englisch 'near vision acuity' bzw. 'reading acuity'). Der Nahvisus, der in Bezug auf das Lesen von Text bestimmt wird, wird auch als Lesevisus bezeichnet. Die Erfindung betrifft Verbesserungen an einer Vorrichtung zur Bestimmung des Nahvisus beim Lesen von Text (= Lesevisus) bzw. Erkennen von Graphiken durch einen Probanden, mit einer Präsentationsfläche, auf der eine Anzahl von Texten (Graphiken) verschiedener Größe darstellbar sind, und einem Mikrofon zum Aufnehmen von Äußerungen des Probanden beim Ablesen der Texte (Erkennen der Graphiken), und mit einer Rechnereinrichtung, die dazu eingerichtet ist, anhand der Mikrofon-Aufnahmen den Ablesevorgang zu überwachen und in Abhängigkeit von der verwendeten, noch lesbaren Schriftgröße einen Nahvisus bzw. Lesevisus des Probanden zu bestimmen.

Vorrichtungen dieser Art sind grundsätzlich bekannt. Hierbei wird der Person, deren Visus bestimmt werden soll, (der Testperson/dem Probanden) eine Graphik oder graphisch dargestellte Symbole, Buchstaben oder Text optisch präsentiert und geprüft, ob diese oder bestimmte Merkmale zutreffend erkannt werden. Dies geschieht für eine Folge gleichartiger Graphiken (bzw. Texte) verschiedener Größe; je geringer die Größe ist, die gerade noch erkennbar ist, desto besser ist der Visus des Probanden. Hierbei werden regelmäßig mehr oder weniger genormte graphische Symbole oder Schriftzeichen verwendet, insbesondere Radner-Lesetafeln, Tafeln mit verschieden orientierten Landolt-C-Symbolen oder die Buchstabentafel nach Snellen. Vorrichtungen zur Unterstützung der Visus-Bestimmung sind beispielsweise in US 6,422,700 und US 6,663,241 beschrieben. Eine Beschreibung der Radner-Lesetafeln und der Bestimmung des Nahvisus bzw. Lesevisus mithilfe dieser Tafeln finden sich z.B. in den Artikeln von W. Radner et al.: "Eine neue Lesetafel zur gleichzeitigen Bestimmung von Lesevisus und Lesegeschwindigkeit", Klin Monatsbl Augenheilkd (1998) 213:174-181; sowie von E. Stifter et al.: "Reliability of a standardized reading chart system: variance component analysis, test-retest and inter-chart reliability", Graefe's Arch Clin Exp Ophthalmol (2004) 242:31-39.

In US 2006/ 0078858 A1 ist ein Verfahren zum Bestimmen des Visus einer Person offenbart, bei welchem die Person eine Gruppe von ihr gezeigten Worten liest und die Lesegeschwindigkeit bestimmt und als Grundlage der Visusbestimmung verwendet wird. Ein Abstand zwischen der Person und der Wortgruppe wird gemessen und bei der Visusbestimmung berücksichtigt. In US 5,568,209 ist ein Sehtestgerät beschrieben, bei dem ein bestimmter Abstand zu der Tafel eingestellt werden kann.

Bekannte Vorrichtungen zur Visusbestimmung stellen auf einzelne Symbole ab, jedoch wird die für den Lesevorgang spezifische Lesedistanz bei der Bestimmung der Sehschärfe (Visus, insbesondere Nahvisus bzw. Lesevisus) nicht berücksichtigt, oder es wird eine bestimmte Lesedistanz vorgegeben, die der Proband einzuhalten hat. Während einerseits die Verwendung verschiedenartiger Lesetafeln und andererseits die Außerachtlassung des subjektiven Leseabstands bei der praktischen Prüfung des Visus meist unproblematisch ist, ergibt sich ein Bedarf nach vergleichbaren Messwerten in wissenschaftlichen Studien und für technische Zwecke, wie z.B. bei vergleichenden Studien von Verfahren, die den Nahvisus bzw. Lesevisus verbessern sollen (z.B. multifokale intraokulare Linsen (MF-IOL), spezielle Nah- bzw. Lesevisus verbessernde Ablationsverfahren der refraktiven Chirurgie ('laser-assisted presbyopia reversal' bzw. "Presby-LASIK) und introcorneale Implantate. Diese Verfahren zielen in erster Linie auf eine Verbesserung des Nahvisus bzw. Lesevisus ab, weshalb hier für eine Vergleichbarkeit der Ergebnisse - und damit für die Beurteilung des jeweiligen Verfahrens - eine Visusbestimmung unter realistischen Bedingungen (z.B. Lesen in frei wählbarem, subjektiv angenehmem Leseabstand) von grundlegendem Wert ist. Eine vergleichbare Visusbestimmung sieht daher die Verwendung standardisierter Texte/Graphiken unter Berücksichtigung des Leseabstands vor; darüber hinaus ist auch eine standardisierte Beleuchtung bei Auflichtvorlagen bzw. Einstellung von Leuchtdichte, Leuchtdichtekontrast und Farbe bei Displays von Bedeutung.

Es ist eine Aufgabe der Erfindung, eine Vorrichtung zu schaffen, die die Erfassung eines objektiven Nahvisus bzw. Lesevisus (wobei die Wahl des jeweiligen subjektiv angenehmen Leseabstandes dem Probanden überlassen wird) und der Lesegeschwindigkeit unter definierten Bedingungen ermöglicht, insbesondere unter Berücksichtigung der Beleuchtungsstärke, der Farbtemperatur des Lichtes bei Auflichtvorlagen bzw. Leuchtdichte, Leuchtdichtekontrast und Farbe bei Displays und des Winkels der Lesefläche. Diese Aufgabe wird von einer Vorrichtung gelöst, welche zur Bestimmung des Visus beim Lesen von Text durch einen Probanden, mit einer Präsentationsfläche, auf der eine Anzahl von Graphiken mit Text verschiedener Schriftgröße darstellbar ist, und einem Mikrofon zum Aufnehmen von Äußerungen des Probanden beim Ablesen der Texte ausgestattet ist, sowie mit einer Rechnereinrichtung, die dazu eingerichtet ist, aus den Mikrofon-Aufnahmen Beginn und Ende des Ablesevorgangs abzuleiten und daraus einen distanzkorrigierten Nahvisus bzw. Lesevisus des Probanden zu bestimmen.

Die genannte Aufgabe wird von einer Vorrichtung gemäß dem Anspruch 1 gelöst. Die Erfindung sieht ein Mittel zum Messen des vom Probanden frei wählbaren Leseabstands des Probanden von dem dargestellten Text (der dargestellten Graphik) auf der Präsentationsfläche (z.B. hochauflösendes LCD-Display oder Lesetafeln bzw. Grafiken) vor, wobei die Rechnereinrichtung dazu eingerichtet ist, unter Verwendung des so gemessenen Leseabstandes den Nahvisus bzw. Lesevisus auf einen distanzkorrigierten Wert zu korrigieren, vorzugsweise unter Verwendung einer photogramm-

metrischen Einrichtung als Mittel zum Messen des Leseabstands.

Durch diesen Ansatz wird die gestellte Aufgabe auf einfache Weise gelöst. Gemäß der Erfindung wird ein Leseabstand nicht vorgegeben, sondern der Proband kann diesen frei wählen und auch während der Visusbestimmung ändern, ohne dass dadurch der Messvorgang beeinträchtigt wird. Erst dadurch, dass der Proband den Leseabstand frei wählen kann, auch verschieden für verschiedene Schriftgrößen, ist eine realistische, dem wahren Leben entsprechende Messung des Lesevisus ermöglicht.

Bevorzugte Ausführungsformen der Erfindung haben entweder ein mit der Präsentationsfläche fest verbundenes Beleuchtungssystem mit zumindest einem Beleuchtungsmittel und einem Lichtmesser zum Messen der im Bereich der Texte/Graphiken herrschenden Beleuchtung (im Sinne einer Leuchtdichte oder Beleuchtungsstärke), sowie einem Regelkreis zum Einstellen der von dem Beleuchtungsmittel ausgehenden Lichtstärke über den Lichtmesser auf einen gewünschten Wert der Beleuchtung, mit der Möglichkeit der Verwendung verschiedener Ausprägungen von Blendung (Glare) die sich dadurch auf den Texten/Graphiken einstellt), oder ein Display mit höchstens 0,166 mm Punktabstand mit computergesteuerter Einstellung von Leuchtdichte, Leuchtdichtekontrast und Farbe, mit der Möglichkeit der Verwendung verschiedener Ausprägungen von Blendung (Glare). Der Lichtmesser kann in vorteilhafter Weise ein in oder an der Präsentationsfläche befindlicher Lichtsensor sein.

Die Berechnung des distanzkorrigierten Lesevisus kann auf einfache Weise erfolgen, nämlich aufgrund einer mathematischen Beziehung (sh. die weiter unten angegebene Gleichung) unter Verwendung des Leseabstands und des Logarithmus der Schriftgröße eines für den Probanden noch lesbaren Textes beruht. Hierbei kann gegebenenfalls auch der Neigungswinkel des Leseguts mathematisch berücksichtigt werden.

Zusätzlich kann der Lesevorgang für den Probanden angenehmer gestaltet werden, wenn die Präsentationsfläche um eine zur Rechts-Links-Achse des Probanden parallele Nickachse zum Probanden hin neigbar ist.

Des Weiteren kann die Rechnereinrichtung zusätzlich dazu eingerichtet sein, aus den Mikrofon-Aufnahmen Beginn und Ende des Ablesevorgangs abzuleiten und daraus in Abhängigkeit von der Länge des jeweils abgelesenen Textes eine Lesegeschwindigkeit zu errechnen.

Das Mittel zum Messen des Leseabstandes kann vorteilhafter Weise eine photogrammetrische Einrichtung sein, die dazu eingerichtet ist, die Position einer Referenzstelle am Gesicht des Probanden, vorzugsweise im Bereich der Augen, zu bestimmen, die in Folge in Beziehung zur Position der Präsentationsfläche zur Berechnung des Leseabstands gesetzt werden kann. Diese Gesichtstelle, z.B. die Nasenwurzel oder der Mittelsteg eines Brillengestells, kann direkt erfasst werden oder mittels einer Marke oder Farbflecks hierfür gekennzeichnet werden.

Bei Verwendung eines Displays entfällt die Beleuchtungseinrichtung. Die Marke oder Farbfleck kann dann z.B. in Tagesleuchtfarbe ausgeführt und während des Messvorganges mit geeigneten UV-LED's beleuchtet sein.

Zur Unterstützung des den Messvorgang leitenden Personals ist es vorteilhaft, wenn die Rechnereinrichtung bzw. ein dort ablaufendes Softwareprogramm zur Erfassung, Speicherung, statistischen Ausarbeitung und zum Ausdrucken der ermittelten Ergebnisse geeignet ist. Außerdem kann bei Auflichtvorlagen eine Einrichtung zum automatischen Wechseln der dem Probanden angebotenen Texte/Graphiken vorgesehen sein. Der Vorlagenwechsel am Display erfolgt computergesteuert. Die Erfindung samt weiterer Vorzügen wird im Folgenden anhand zweier bevorzugter Anwendungsbeispiele näher erläutert, die in den beigefügten Figuren illustriert sind.

Die Figuren zeigen

Fig. 1 eine Leseplatz-Anordnung für Auflichtvorlagen (SRD-1) gemäß der Erfindung in einer Seitenansicht von rechts,

Fig. 1a eine Leseplatz-Anordnung mit Display (SRD-2) gemäß der Erfindung in einer Seitenansicht von rechts,

Fig. 2 die Anordnung der Fig.1 in einer Vorderansicht,

Fig. 2a die Anordnung der Fig.1a in einer Vorderansicht,

Fig. 3 ein schematisches Blockdiagramm der Anordnung der Fig.1,

Fig. 3a ein schematisches Blockdiagramm der Anordnung der Fig.1a, und

Fig. 4 bis 6 verschiedene Dialogfenster der zugeordneten Steuer- und Messsoftware.

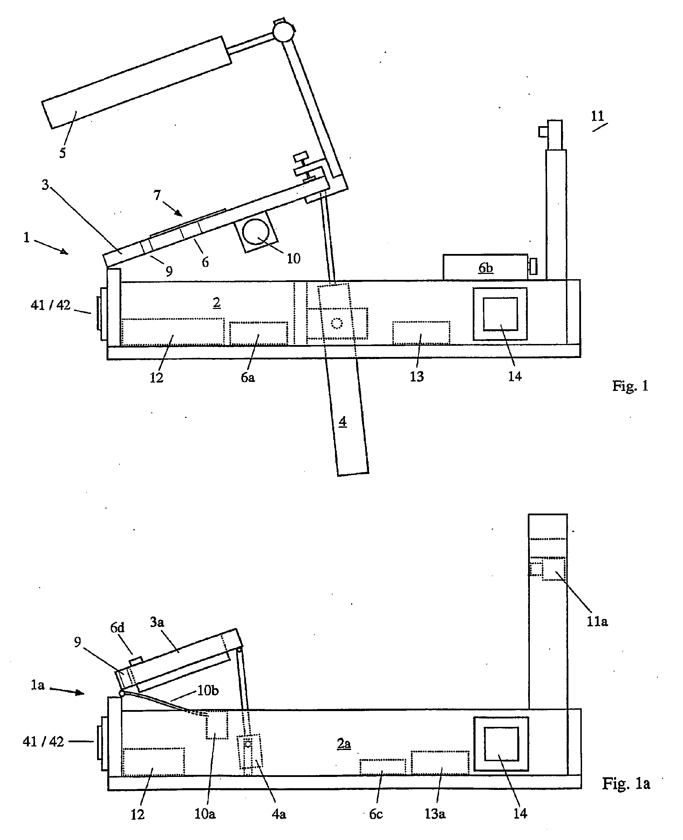

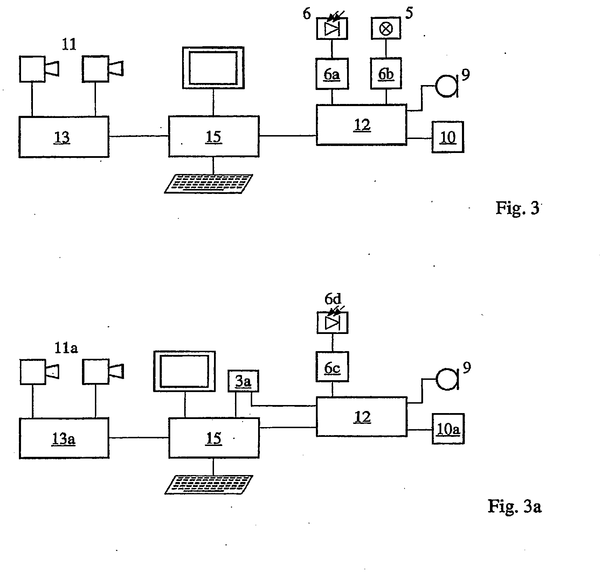

Das nachfolgende Ausführungsbeispiel betrifft eine Anordnung SRD-1 bzw. SRD-2, die nach ihrem Entstehungsort auch als 'Salzburg Reading Desk' bezeichnet wird und die zur Bestimmung des distanzkorrigierten Lesevisus unter Einbeziehung des photogrammetrisch gemessenen mittleren Leseabstandes und gleichzeitiger Messung und Protokollierung der Lesegeschwindigkeit, des Lesewinkels und der standardisierten Beleuchtung bzw. bei Verwendung eines Displays mit unterschiedlicher Leuchtdichte, Leuchtdichtekontrast und Farbe, mit der Möglichkeit der Verwendung verschiedener Ausprägungen von Blendung (Glare) eingesetzt wird. Die Erfindung ist jedoch nicht auf dieses Ausführungsbeispiel SRD beschränkt. Die Anordnung besteht aus einem Lesepult-artigen Testplatz, der in Fig.1 bzw.1a gezeigt ist, sowie einem Steuer-Rechner als Steuereinrichtung, beispielsweise mittels eines Personal Computers oder Laptop-Computers (Bezugszeichen 15 in Fig. 3 bzw. 3a) mit geeigneter Steuer- und Mess-Software realisiert.

Auf dem Testplatz der Anordnung SRD-1 bzw. SRD-2 können Lesekarten als Auflichtvorlagen oder Durchlichtvorlagen am Display einer Testperson angeboten werden. Jede Vorlage hat einen standardisierten Text mit normierter Schriftgröße und vorzugsweise standardisiertem Inhalt. Die Testperson liest den Text von der Vorlage ab (bzw. versucht es), und aufgrund dessen wird ein (vorläufiger) Lesevisus bestimmt. Die Vorrichtung weist zusätzlich eine Einrichtung zum Bestimmen des Leseabstands auf, vorzugsweise mittels Kameras realisiert. Der Leseabstand wird - vorzugsweise während des Lesevorgangs - gemessen, und aus dem Leseabstand und der Schriftgröße des jeweiligen Textes wird ein distanzkorrigierter Lesevisus berechnet. Die Vorrichtung kann außerdem eine kontrollierte Beleuchtung sowie ein Mikrofon aufweisen, das zum Messen der Lesedauer verwendet werden kann.

Als Lesetafeln bzw. Durchlichtvorlagen werden die eingangs erwähnten Radner-Lesetafeln verwendet. Jeder Absatz dieser Tafeln enthält einen (deutschen) Satz, der aus 14 Worten besteht, die auf 3 Zeilen angeordnet sind. Die Zeichengrößen der Lesetafeln sind entsprechend einer geometrischen Reihe abgestuft, und zwar mit einem Faktor $10^{0,1}$. Den Lesetafeln sind LogRAD-Werte zugeordnet, die dem (skalierten) Logarithmus der Zeichengröße entsprechen; die LogRAD-Werte laufen folglich linear, beispielsweise von 0,9 (größte Schrifttype) bis -0,2 (kleinste Schrift) für einen Leseabstand von 40 cm. Weitere Informationen zu den Radner-Tafeln sind den bereits genannten Artikeln von W. Radner *et al.* und E. Stifter *et al.* zu entnehmen.

Es ist allerdings für den Fachmann unmittelbar einleuchtend, dass auch andere Vorlagen verwendet werden können; diese Vorlagen können geeignete Symbole oder Graphiken verschiedenster Art enthalten, wie z.B. Ziffern Zahlen, E-Haken, Landolt-Ringe, Schriftzeichen anderer Sprachen, Musiknoten, Landkarten, Stadtpläne, Fahrpläne, Bilder für Kinder, die unter anderem in der Ausführung SRD-2 auf dem zur Präsentation dienendem Display unter anderem in verschiedenen Kontraststufen bzw. Farben eingeblendet werden können, mit der Möglichkeit der Verwendung verschiedener Ausprägungen von Blendung (Glare).

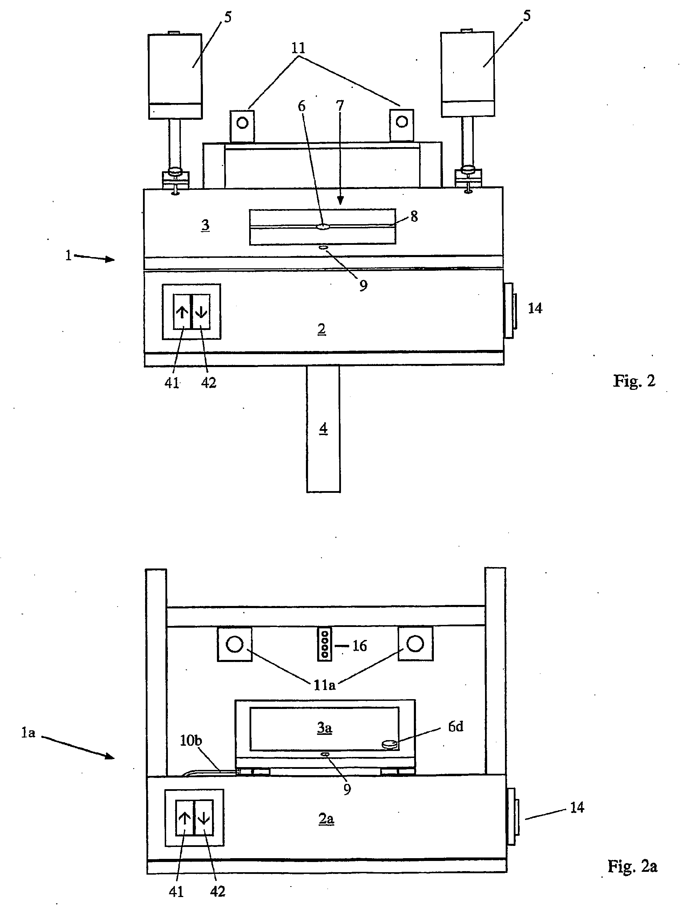

Bei Verwendung eines Displays zur Präsentation der Zeichen können zusätzlich Leuchtdichte, Leuchtdichtekontrast und Farben als Untersuchungskriterien herangezogen werden, mit der Möglichkeit der Verwendung verschiedener Ausprägungen von Blendung (Glare). Bezugnehmend auf Fig.1 und 2 weist die Anordnung SRD-1 ein Gehäuse 1 auf, dessen Grundkonstruktion ein liegender, durch einen Steg unterteilter Rahmen 2 ist, auf dem eine aufklappbare flache Platte 3 als Präsentationsfläche befestigt ist, die in etwa die Hälfte des Gehäuses 1 bedeckt An dem Steg ist mittig ein handelsüblicher Spindelmotor 4 befestigt, mit dessen Hilfe der Proband den Winkel der Lesefläche zwischen 0° und 40° selbst einstellen kann. Hierzu befinden sich zwei Wippschalter 41, 42 auf der dem Probanden zugewandten Seite des Gehäuses. Der Spindelmotor 4 ragt in der gezeigten Ausführungsform nach unten durch die Auflagefläche hinaus.

An der hinteren Kante der Lesefläche 3 sind bei der Anordnung SRD-1 zwei handelsübliche Lampen 5 (sogenannte Reproleuchten), die eine flimmerfreie Beleuchtung (z.B. mit Gleichstrom- oder Hochfrequenz-Versorgung) liefern, hier zu je 18 Watt Leistung und einer Licht farbe von 5400 K befestigt. Die Helligkeit der Lampen 5 auf der Lesefläche wird mittels eines in der Lesefläche montierten Messkopfes 6 eines Luxmeters 6a, eines Steuergeräts 6b und des Steuer-Rechners 15 konstant gehalten. Der Benutzer (Untersuchungsleiter) stellt die gewünschte Beleuchtungsstärke, beispielsweise 500 Lux ($\pm$10 Lux), am Steuerungs-Rechner ein.

In einer alternativen Realisierung kann bei der Anordnung SRD-1 die Lichtmessung durch Messung der Leuchtdichte (in cd/m2) am Ort der Lesetafeln mittels eines über der Lesefläche 3 angebrachten, auf die Lesefläche gerichteten Sensors (nicht gezeigt) erfolgen. In einer anderen, vereinfachten Variante kann die Helligkeit auch mit Hilfe eines (nicht gezeigten) Dimmers anstelle des Steuergeräts 6a auf die jeweils gewünschte Leuchtdichte bzw. Beleuchtungsstärke eingestellt werden.

Auf der Oberseite der Lesefläche sind bei der Anordnung SRD-1 eine Anzahl von Lesetafeln 7, beispielsweise die einzelnen Absätze der standardisierten Radner-Lesetafeln, befestigt, beispielsweise mittels einer horizontal (von rechts nach links) verlaufenden Ringbuchartigen Befestigung 8, die ein Umblättern der einzelnen Tafeln nach unten (d.h. in Richtung kleinerer Schriftgröße) zulässt, wobei jede Schriftgröße auf einem eigenen Blatt dargestellt ist Vom Probanden werden die Blätter im Zuge der Untersuchung weitergeblättert. Aufgabe des Probanden ist es, die auf den Lesetafeln präsentierten Texte nacheinander laut vorzulesen. Der distanzkorrigierte Lesevisus wird aus der gelesenen Schrifthöhe und dem gemessenen mittlerem Leseabstand berechnet. Zusätzlich wird die Lesedauer gemessen.

Dieses mit Ringrücken gebundene "Heft" ist derart gestaltet, dass bei der Anordnung SRD-1 der in die Lesefläche eingelassene Messkopf 6 des Luxmeters genau im Zentrum des aufgeschlagenen Heftes von der Beleuchtung 5 beschienen wird und so eine exakte Beleuchtung (Leuchtdichte bzw. Beleuchtungsstärke) der Lesefläche-gewährleistet ist.

Bezugnehmend auf Fig. 1a und 2a weist die Anordnung SRD-2 ein Gehäuse 1a auf, dessen Grundkonstruktion ein liegender Rahmen 2a ist, auf dem ein aufklappbares Display 3a als Präsentationsfläche befestigt ist. Weiters ist ein an dem Display 3a anliegender Lichtmesser 6d vorgesehen, welcher als auf das Display 3a gerichteter Lichtsensor ausgebildet ist. Am Display 3a können mit dem angeschlossenen PC 15 Leuchtdichte, Leuchtdichtekontrast, Farbe und der Vorlagenwechsel gesteuert werden, mit der Möglichkeit der Verwendung verschiedener Ausprägungen von Blendung (Glare).

Zwischen den Kameras 11a befindet sich eine geeignete Anzahl von senkrecht angeordneten UV-LED's 16, die während des Messvorganges die Messmarke oder Farbfleck beleuchten.

Im Gehäuse ist ein handelsüblicher Kettenschubantrieb 4a befestigt, mit dessen Hilfe der Proband die Neigung der Lesefläche zwischen 0° und 90° selbst einstellen kann. Hierzu befinden sich zwei Wippschalter 41, 42 auf der dem Probanden zugewandten Seite des Gehäuses. Der Kettenschubantrieb 4a liegt in der gezeigten Ausführungsform zur Gänze im Gehäuse. Der Lese- und Messvorgang entspricht der Anordnung SRD-1.

In die Lesefläche bzw. in den Rahmen des Displays integriert ist weiters ein Mikrofon 9, das an einen (nicht gezeigten) akustischen Schwellwertschalter angeschlossen ist. Das Mikrofon dient dazu, die Sprache des Probanden aufzuzeichnen und damit die jeweilige Lesedauer des Probanden genau festzustellen. Daraus kann die Steuer/Messsoftware die Lesegeschwindigkeit in Worten pro Minute berechnen. Hierzu wird mittels dieses akustischen Schwellwertschalters bestimmt, wann der Proband spricht. Eine Sprechpause, die über einen längeren Zeitraum anhält, beispielsweise über einige Sekunden, (denn kurze Pausen können auf Sprechpausen wie Atemholen oder stimmlose Laute zurückgehen) wird als Ende des Lesevorgangs interpretiert.

Anstelle des akustischen Schwellwertschalters, oder in Ergänzung dazu, kann eine Signal-Taste vorgesehen sein, mit der der Proband (z.B. im Falle eines stummen oder sprechbehinderten Probanden) die Lesezeit signalisiert, beispielsweise durch Drücken der Signal-Taste jeweils bei Beginn und Ende des Lesens eines Textes. Durch Verwendung einer Signaltaste kann außerdem der Messvorgang im Wesentlichen ohne Kontrollperson ablaufen, in Hinblick auf eine Automatisierung des Untersuchungablaufes. Außerdem kann ein optisches Signal (Signallampe) vorgesehen sein, um insbesondere einem hörbehinderten Probanden den Beginn des Messvorgangs anzuzeigen. Dieses optische Signal kann somit als Ergänzung des akustischen Start-Signals des Lesevorgangs dienen.

An der Lesefläche, vorzugsweise auf ihrer Unterseite, ist ein handelsüblicher Neigungswinkelmesser 10 bekannter Art montiert. Der Winkel der Lesefläche kann auch mit einer flexiblen Welle 10b auf einen Winkelsensor 10a übertragen werden.

An der der Lesefläche gegenüberliegenden Seite des Gehäuses sind auf einer Bühne zwei Videokameras 11, 11a hoher Auflösung (z.B. 1,3 MPx) angebracht. Die Kameras sind beispielsweise AXIS 206M der Firma Axis Communications AB (Schweden) oder Kameras der Firma "The Imaging Source", USA-DE. Mithilfe dieser Videokameras 11, 11a kann der Leseabstand (d.i. der Abstand eines Referenzpunktes im Gesicht des Probanden, wie z.B. Nasenwurzel, oder Brillengestell, von den Lesetafeln) bestimmt werden. Eine Marke, z.B. in Form eines farbigen Klebepunktes in einer Tagesleuchtfarbe mit 12 mm Durchmesser, wird am Beginn der Untersuchung auf die Nasenwurzel des Probanden oder auf den Mittelsteg eines Brillengestells geklebt. Mithilfe der Videokameras wird die Position der Marke bestimmt, und daraus wird mittels Photogrammetrie der Abstand der Marke zur gelesenen Zeile in Abhängigkeit vom eingestellten Lesewinkel unter Einbeziehung der geometrischen Anordnung der Videokameras (gegenseitiger Abstand, Höhe, Ausrichtung) berechnet. Die Videokameras 11, 11a befinden sich hinter dem Lesetisch 3, 3a in einer wohlbestimmten Anordnung relativ zu der Präsentationsfläche (in der gezeigten Ausführungsform SRD-1 72,7 cm von der Vorderkante der Fläche 3 entfernt und 51 cm über der Grundplatte, in einem gegenseitigen Abstand von 21 cm) und sind so ausgerichtet, dass beide den Raumbereich erfassen, den der Kopf des Probanden einnimmt; vorzugsweise liegen die optischen Hauptachsen der Kameras in einer horizontalen Ebene. Aus den Bildkoordinaten des Objektpunktes, der die Marke auf der Nasenwurzel bzw. am Brillengestell repräsentiert, werden aus den beiden Kamera-Bildern die räumlichen Koordinaten der Marke und in der Folge der Abstand der Marke von dem zu lesenden Text berechnet. Hierzu wird in jedem Bild der Objektpunkt, der durch die besondere Farbe der Marke gekennzeichnet ist, gesucht. Der Mittelpunkt des gefundenen Farbbereichs wird als Ergebniskoordinate verwendet. Aufgrund der Ergebniskoordinaten beider Kamera-Bilder wird nach bekanntem Stereobild-Verfahren die Position der Marke in Bezug auf die Kamerapositionen bestimmt. Daraus wird aufgrund der bekannten Lagebeziehung zwischen den Kameras 11, 11a und den Lesetafeln 7 bzw. dem Display 3a, deren Position bei Kenntnis des mit dem Neigungsmesser 10 bzw. 10a mit oder ohne flexibler Welle 10b gemessenen Neigungswinkels genau bekannt ist, der jeweilige Leseabstand abgeleitet.

Die Erfindung ist allerdings nicht an die photogrammetrische Messung des jeweiligen, vom Probanden frei wählbaren Leseabstandes gebunden; vielmehr kann diese auch auf eine andere geeignete Weise bestimmt werden, beispielsweise akustisch mittels Ultraschall.

Der Bereich der Lesetafel 7 bzw. dem Display 3a, falls gewünscht auch die gesamte Vorrichtung SRD-1 bzw. -2, kann zusätzlich von einem Guckkasten-artigen Gehäuse (nicht gezeigt) umgeben werden, die auch als tunnelförmige Abdeckung realisiert sein kann. Auf diese Weise wird eine Dejustierung der Kameras und der Beleuchtung vermieden, außerdem gestattet dies die Verwendung des Geräts unabhängig von Umgebungslicht, insbesondere hinsichtlich Stärke, Farbe und Frequenz des Störlichts. Auch Störungen der Videokameras 11, 11a, beispielsweise durch Reflexe auf der Kleidung des Probanden, werden so vermieden.

Wie auch aus den Blockdiagrammen der Fig. 3, 3a zu ersehen ist, ist eine Datenschnittstelle 12 vorgesehen, die über analoge Eingänge und einen USB-Ausgang mit dem Steuer-Rechner 15 kommuniziert, auf dem die Mess- und Steuersoftware abläuft; die Datenschnittstelle 12 ist beispielsweise mithilfe eines DAQ-Moduls USBDAQ-9100MS der Adlink Technology Inc. (Taiwan/USA) implementiert. Außerdem sind im Inneren des Gehäuses alle erforderlichen Netzgeräte zur Niederspannungsversorgung (nicht gezeigt) sowie die Stromversorgung und der Anschluss ans elektrische Netz für den Spindelmotor 4 bzw. Kettenschubantrieb 4a untergebracht. Über die Datenschnittstelle werden die analogen Daten von Neigungswinkelmesser, Luxmeter 6a bzw. Leuchtdichtemesser 6c und akustischem Schwellwertschalter eingespeist und über einen USB-Ausgang an den PC ausgegeben. Im Rahmen 2, 2a ist ein Ethernet-Switch 13 eingebaut, der ein LAN realisiert, über das die beiden Videokameras 11 mit dem PC kommunizieren, bzw. bei Verwendung von Firewire-Kameras 11a ein Firewire-HUB 13a.

Die Einbauten unter der Lesefläche sind durch ein metallisches Gitter abgeschirmt. Von dem Testplatz führen somit drei Anschlussleitungen weg, nämlich ein Netzkabel (z.B. 230 V) zur Stromversorgung am üblichen Stromnetz, eine USB-Leitung sowie ein Ethernet- oder Firewire-Verbindung zum Steuer-Rechner 15. Die Stromversorgung der einzelnen Komponenten sowie der Spindelmotor 4 bzw. Kettenschubantrieb 4a sind in Fig. 3, 3a nicht dargestellt.

Alle Geräte werden mit einem zentralen Netzschalter 14 von außen ein- bzw. ausgeschaltet, der beispielsweise an der Gehäuse-Rückseite oder seitlich am Gehäuse angebracht ist. Nach dem Einschalten der Anordnung SRD-1 wird abgewartet, bis die Beleuchtung (Leuchtdichte bzw. Beleuchtungsstärke) durch die Lampen 5 so stabil ist (dies kann je nach Typ der Lampen mehrere Minuten bis eine halbe Stunde dauern), dass mit dem Beleuchtungsregelkreis die jeweils festgelegte Beleuchtungsstärke eingestellt werden kann. Beim Einschalten der Anordnung SRD-2 muss ebenfalls gewartet werden, bis sich die Leuchtdichte des Displays 3a stabilisiert.

Vor der Untersuchung wird der Proband aufgefordert, einen für ihn angenehmen Lesewinkel über die Tasten 41, 42 einzustellen; hierbei wird als Grundeinstellung ein Winkel von 20° vorgegeben, wobei bei der Anordnung SRD-1 der Winkel je nach der jeweiligen Lesegewohnheit des Probanden zwischen 0° und 40°, bei der Anordnung SRD-2 zwischen 0° und 90° frei wählbar ist.

Eine Mess- und Steuersoftware kontrolliert den Ablauf der Visusbestimmung. Sie ist vorteilhafter Weise so gestaltet, dass der Untersuchungsleiter per einfachem Tastendruck bzw. Mausklick durch die Untersuchung geführt werden kann, wobei einerseits auf keine der notwendigen Eingaben vergessen werden kann und andererseits eine automatische Abspeicherung bzw. der Ausdruck des Ergebnisses erfolgt. Vorteilhafter Weise wird für den gesamten Ablauf eine Schritt-für-Schritt-Führung vom Computer optisch und/oder akustisch ausgegeben. Dadurch wird die Einschulungszeit des Bedienpersonals reduziert und zusätzlich werden Fehler im Laufe der Visusbestimmung vermieden. In einer weiteren Realisierung wird die automatische ablaufende Untersuchung ohne notwendige Anwesenheit eines Untersuchungsleiters ermöglicht.

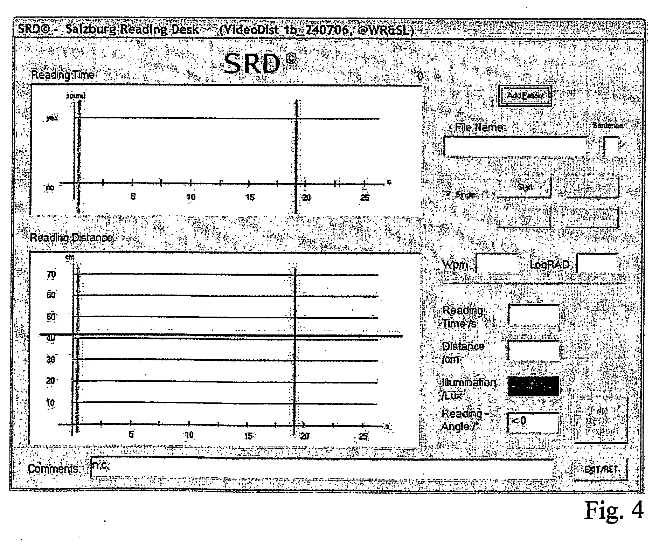

Die Mess- und Steuersoftware läuft auf einem PC mit Bildschirm, Maus und Tastatur ab und wird vorzugsweise getrennt von der Vorrichtung SRD-1 gestartet, bzw. kann in einer weiteren Realisierung automatisiert ohne notwendige Anwesenheit eines Untersuchungs-leiters ablaufen. Bei Verwendung der Anordnung SRD-2 erfolgt der Start gleichzeitig. Zu Beginn führt die Software der Reihe nach folgende Funktionen aus: Prüfung der Integrität der Programmdatei, Darstellung des Vorbereitungsfensters am Bildschirm, Einlesen der Geräte- und Messparameter aus einer Parameterdatei, Starten der Bildübertragung von den beiden Kameras zum PC und Darstellung als zwei Live-Videobilder am Bildschirm (z.B. als Inserts). Sind die beiden Videobilder zu sehen, startet der Untersuchungsleiter bzw. Benutzer den eigentlichen Untersuchungsvorgang durch eine spezifische Eingabe, z.B. mit der Eingabetaste. Das Programm beginnt sofort mit der laufenden Messung der Leuchtdichte bzw. Beleuchtungsstärke und des Neigungswinkels; diese werden im Hauptfenster der Anzeige wie in Fig. 4 gezeigt ausgegeben.

Die Schaltflächen sind vorteilhafter Weise unterschiedlich gestaltet: Schaltfläche mit doppeltem Rahmen werden mit der Eingabetaste aktiviert; Schaltflächen mit einfachem Rahmen können mit der Maus oder mit "hot key" aktiviert werden; Schaltflächen mit grauem Text sind nicht aktivierbar. Dies ermöglicht die Standardbedienung (im Sinne des üblichen Ablaufs der Visusbestimmung) mit nur einer Taste, z.B. der Eingabetaste. Dadurch wird die Gefahr einer Fehlbedienung weitgehend ausgeschlossen, ohne die Flexibilität einzuschränken.

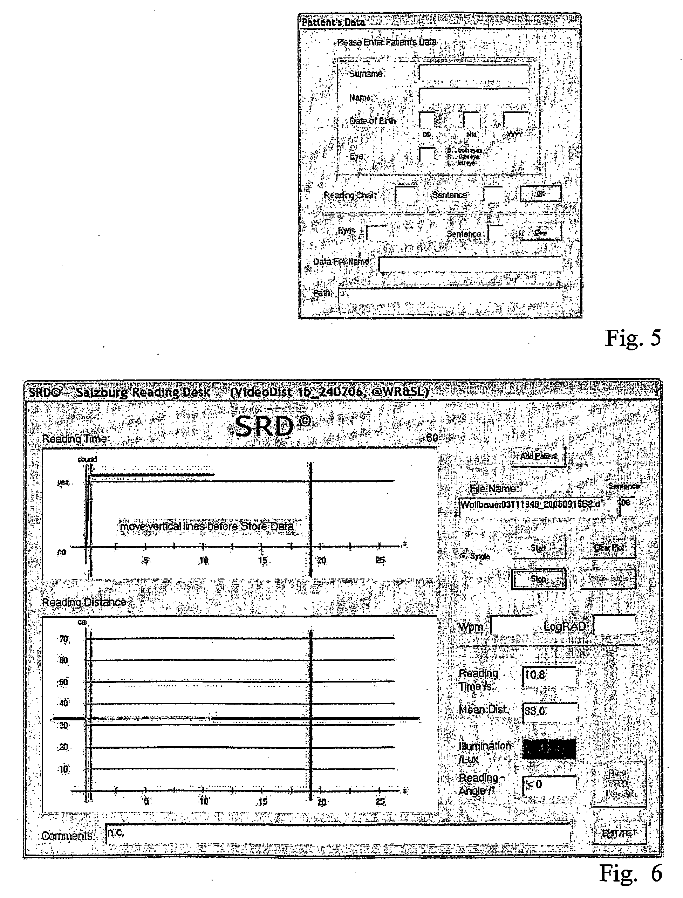

Das Programm erwartet nun die Eingabe Probanden-spezifischer Daten. Nach Aktivieren der Schaltfläche <Add Patient> wird eine in Fig. 5 gezeigte Eingabemaske <Patient's Data> gezeigt; hier werden die Eingabe von <Surname>, <Name>, <Date of Birth>, <Eye>, <Reading Chart> und <Sentence> zwingend verlangt und - wo erforderlich - mit Plausibilitätsüberprüfungen abgesichert.

Vor der Dateneingabe wird mit dem Probanden vereinbart, mit welchem Satz der Radner-Tafeln die Untersuchung beginnt. Jeder Satz ist in einer bestimmten Schrifthöhe wiedergegeben, die der Satznummer entspricht; durch die Wahl der Satznummer wird somit die Schrifthöhe gewählt (bzw. umgekehrt).

Aus diesen Daten wird automatisch ein Dateiname generiert, der das Wiederfinden der Daten ermöglicht; Beispiel: Wolfbauer03111946_20060915B2.dat - zusammengesetzt aus dem Namen und Geburtsdatum (ddmmyyyy) des Probanden, Untersuchungsdatum sowie zwei Kennbuchstaben zur Kennzeichnung des Untersuchungstyps, wobei im Beispiel B2 für beide Augen und Absatz (Lesetafel) Nr. 2 steht.

Nach Beendigung der Eingabe der Probanden-Daten und Bestätigung (z.B. mit <OK>) erscheint wieder das Programm-Hauptfenster.

Mit <Enter> ertönt ein Signal, das den Beginn für den Probanden anzeigt.

Die Ton- und Entfernungsmessung wird mit der Schaltfläche <Start> gestartet und in der Folge im Hauptfenster angezeigt, wie in Fig. 6 dargestellt. Das Programm überprüft das Signal am Ausgang des Tonverstärkers und zeigt seinen Zustand im Zeitdiagramm des SRD Fensters an. Anschließend werden in beiden Videobildern die jeweiligen Bildkoordinaten des Mittelpunkts der Farbmarke festgestellt. Die Farbe der Farbmarke darf im Bild sonst nicht vorkommen. Aus diesen Bildkoordinaten, dem Kameraabstand und dem Abbildungsmaßstab der Kameras errechnet das Programm in einem nächsten Schritt stereophotogrammetrisch die Raumkoordinaten der Position der Farbmarke. Im folgenden Schritt wird der Abstand der Farbmarkenposition von der Lesezeile, deren Koordinaten dem Programm bekannt sind, berechnet. Das Ergebnis wird als Zahlenwert in seiner Ausgabebox und im Abstandsdiagramm des SRD Fensters graphisch dargestellt. Dieser Vorgang wiederholt sich ständig, maximal 25 Sekunden lang, oder wird durch Aktivieren der Schaltfläche <Stop> beendet. Während dieser Prozeduren unterbleibt die Winkel- und Helligkeitsmessung.

Nach Leseende wird der Messvorgang beendet. Sodann wird der Mittelwert der gemessenen Entfernungen errechnet, als Wert in der Ausgabebox angezeigt und im Abstandsdiagramm als horizontale Linie dargestellt.

Durch Ziehen der beiden Messmarken im Akustikdiagramm (die Messmarken laufen in beiden Diagrammen synchron) wird der Zeitbereich auf die tatsächliche Lesezeit eingegrenzt. Gleichzeitig wird der Mittelwert der Entfernungsmesswerte innerhalb dieses Bereiches neu errechnet und als Wert und als Linie im Diagramm dargestellt. Auch die Werte für die Lesegeschwindigkeit und den Lesevisus werden entsprechend dem eingegrenzten Zeitbereich neu berechnet und angezeigt. Erst das Eingrenzen der Lesezeit durch Ziehen der vertikalen grünen bzw. roten Messlinien ermöglicht das Speichern des Datensatzes bzw. es werden folgende errechnete Daten angezeigt: Wpm (Wörter/min), LogRAD, Reading Time, Mean Distance.

Die Lesegeschwindigkeit $v$ (angegeben in Wpm = Wörter pro Minute) wird gemäß

$$v = 60 \times 14 / t$$

berechnet, wobei t die Lesedauer in Sekunden für einen Satz (der eine Länge von 14 Wörtern hat) ist (vgl. den Artikel von W. Radner *et al.*). Der distanzkorrigierte Visus in Form einer LogRAD-Größe wird gemäß

$$\text{LogRAD} = 1{,}2 - 0{,}1 \times SN + \log_{10} (40 / Distmv)$$

aus der Satznummer *SN* der verwendeten Radner-Lesetafel berechnet, wobei *Distmv* der Mittelwert des gemessenen Leseabstandes in cm ist. Der Lesevisus als LogRAD kann in einen Visus nach Snellen gemäß

$$\text{Snellen} = 10^{-\text{LogRAD}}, \quad \text{bzw.:} \quad \text{LogRAD} = -\log_{10} \text{Snellen},$$

umgerechnet werden.

Vor dem Speichern ist es weiters möglich, Kommentare zu den einzelnen Messungen (= Comments), soweit als notwendig erachtet, einzugeben und auch die Angabe, ob das Lesen mit/ohne optische(r) Korrektur (sphärisch, zylindrisch) erfolgt ist.

Alle Messwerte und errechneten Werte einer Untersuchungsreihe werden in einer spezifischen Datei gespeichert, was die spätere Auswertung mit statistischen Methoden ermöglicht. Außerdem wird ein patientenbezogener Report ausgedruckt und/ oder als druckfähige Datei abgespeichert.

Nach dem Speichern springt der Fokus auf <Print SRD Report>, was durchgeführt werden kann, aber nicht muss, denn üblicherweise erfolgt der Ausdruck erst nach dem letzten erfolgreich gelesenen Satz (= Sentence) bzw. gespeicherten Messergebnissen, wobei dann in diesem alle ermittelten Ergebnisse angeführt werden.

Der Fokus springt wieder auf <Start>, wobei im Feld <Sentence> (Absatznummer) der Zähler um 1 erhöht wird. Bei der bei der Anordnung SRD-2 erscheint durch Drücken von <Start> die nächste Vorlage automatisch am Display 3a. Da es nur 14 Absätze gibt, endet die Untersuchungsreihe nach der 14. Absatznummer. Der Fokus springt dann auf <Add Patient>, und es kann der nächste Patient untersucht werden.

Gemessen und angezeigt werden:

- Leseabstand

- Kontrast bzw. Leuchtdichtekontrast

- Helligkeit (z.B. gemessen in Lux oder cd/m$^2$)

- Neigung des Lesepultes (= Lesewinkel)

- Lesedauer der standardisierten Texte (Radner Lesetafeln) pro Textgröße

- Lesedauer der standardisierten Texte (Radner Lesetafeln) über die gesamte Prüfperiode

Berechnet und angezeigt werden:

- Mittlerer Leseabstand über die Prüfperiode pro Textgröße

- Mittlerer Leseabstand über die gesamte Prüfperiode

- Lesevisus nach Prof. Radner (LogRAD) sowie nach Snellen (nur im Patientenreport)

- Lesedauer standardisierter Texte (Radner Lesetafeln) über die gesamte Prüfperiode Lesedauer der standardisierten Texte (Radner Lesetafeln) pro Textgröße

- Lesegeschwindigkeit in Worten pro Minute pro Textgröße

- "Critical Print Size" (d.i. die kleinste Schriftgröße, die noch mit maximaler Lesegeschwindigkeit gelesen werden kann)

Die Erfindung ist selbstverständlich nicht auf die hier gezeigten Ausführungsformen SRD-1, SRD-2 eingeschränkt. So können die Lesetafeln (Radner oder andere) auch maschinell umgeblättert werden, beispielsweise Computer-gesteuert. Anstelle der Lesetafeln können auch Computer-gesteuerte Lesebänder, z.B. wie die "Road Book" - Einrichtung für Motorradfahrer, eingesetzt werden. Statt dessen kann auch ein hochauflösendes Display zur Darstellung jeglicher Art von Texten und Graphiken verwendet werden. Das Display verfügt zusätzlich über essentielle Möglichkeiten bezüglich Einstellung von Leuchtdichte, Leuchtdichtekontrast und Farbe, mit der Möglichkeit der Verwendung verschiedener Ausprägungen von Blendung (Glare). Zusätzlich zur Ausgabe über einen Bildschirm kann auch ein Drucker, vorzugsweise ein Farbdrucker, vorgesehen sein.

**Patentansprüche**

1. Vorrichtung zur Bestimmung des Nahvisus beim Lesen/Erkennen von Texten/Graphiken durch einen Probanden, mit einer Präsentationsfläche (3, 3a), auf der eine Anzahl von Texten/Graphiken (7) in verschiedener Schriftgröße/Größe darstellbar sind, und einem Mikrofon (9) zum Aufnehmen von Äußerungen des Probanden beim Ablesen/ Erkennen der Texte/Graphiken, und mit einer Rechnereinrichtung, die dazu eingerichtet ist, anhand der Mikrofon-Aufnahmen den Lese/Erkennungs-Vorgang zu überwachen und in Abhängigkeit von der verwendeten Schriftgröße/Größe einen Nahvisus des Probanden zu bestimmen, wobei ein Mittel zum Messen des vom Probanden wählbaren Leseabstandes von dem dargestellten Text bzw. der dargestellten Graphik (7) verwendet wird, **dadurch gekennzeichnet, dass** die Recheneinrichtung dazu eingerichtet ist, aufgrund einer mathematischen Beziehung unter Verwendung des so gemessenen Leseabstandes und des Logarithmus der Schriftgröße eines für den Probanden noch lesbaren Textes den Nahvisus auf einen distanzkorrigierten Wert zu korrigieren, wobei das Mittel zum Messen des Leseabstands eine stereophotogrammetrische Einrichtung mit zwei Videokameras (11, 11a) ist, die dazu eingerichtet ist, die Position einer Referenzstelle am Gesicht des Probanden zu bestimmen, die in Folge in Beziehung zur Position der Präsentationsfläche zur Berechnung des Leseabstands gesetzt werden kann.

2. Vorrichtung nach Anspruch 1, **gekennzeichnet durch** ein mit der Präsentationsfläche (3) fest verbundenes Beleuchtungssystem mit zumindest einem Beleuchtungsmittel (5) und einem Lichtmesser (6) zum Messen der im Bereich der Texte/Graphiken herrschenden Beleuchtung, sowie einem Regelkreis zum Einstellen der von dem zumindest einen Beleuchtungsmittel ausgehenden Lichtstärke über den Lichtmesser (6) auf einen gewünschten Wert der Beleuchtung.

3. Vorrichtung nach Anspruch 2, **dadurch gekennzeichnet, dass** der Lichtmesser (6) ein in der Präsentationsfläche (3) befindlicher Lichtsensor ist.

4. Vorrichtung nach einem der Ansprüche 1 bis 3, **dadurch gekennzeichnet, dass** sie einen Leuchtdichtemesser (6+6c) aufweist.

5. Vorrichtung nach einem der Ansprüche 1 bis 4, **dadurch gekennzeichnet, dass** sie ein hochauflösendes Display (3a) aufweist, dessen Leuchtdichte und/oder Leuchtdichtekontrast und/oder Farbe bzw. Schrift- oder Zeichengrößen computergesteuert mess- bzw. adjustierbar sind.

6. Vorrichtung nach Anspruch 5, **dadurch gekennzeichnet, dass** ein am Display (3a) anliegender Lichtmesser (6d) vorgesehen ist, welcher als auf das Display (3a) gerichteter Lichtsensor ausgebildet ist.

7. Vorrichtung nach einem der Ansprüche 1 bis 6, **dadurch gekennzeichnet, dass** die Präsentationsfläche um eine zur Rechts-Links-Achse des Probanden parallele Nickachse zum Probanden hin neigbar ist.

8. Vorrichtung nach einem der Ansprüche 1 bis 7, **dadurch gekennzeichnet, dass** die Rechnereinrichtung zusätzlich dazu eingerichtet ist, aus den Mikrofon-Aufnahmen Beginn und Ende des Ablesevorgangs abzuleiten und daraus in Abhängigkeit von der Länge des jeweils abgelesenen Textes eine Lesegeschwindigkeit zu errechnen.

9. Vorrichtung nach einem der Ansprüche 1 bis 8, **dadurch gekennzeichnet, dass** die Rechnereinrichtung bzw. ein dort ablaufendes Softwareprogramm zur Erfassung, Speicherung, statistischen Ausarbeitung und zum Ausdrucken der ermittelten Ergebnisse geeignet ist.

10. Vorrichtung nach einem der Ansprüche 1 bis 9, **gekennzeichnet durch** eine Einrichtung zum maschinellen/computergesteuerten Wechseln der dem Probanden angebotenen Texte/Graphiken.

11. Vorrichtung nach Anspruch 10, **dadurch gekennzeichnet, dass** die dem Probanden angebotenen Texte/Graphiken auf einem Bildschirm dargestellt werden.

12. Vorrichtung nach einem der Ansprüche 1 bis 11, **dadurch gekennzeichnet, dass** die dem Probanden angebotenen Texte/Graphiken in Form von Lesetafeln oder auf einem Leseband realisiert sind.


**Claims**

1. An apparatus for determining the near vision acuity during the reading/recognition of texts/graphics by a test person, comprising a presentation surface (3, 3a) on which a number of texts/graphics (7) in different font sizes/sizes can be displayed, and a microphone (9) for recording statements made by the test person while reading/recognizing the texts/graphics, and a computer device which is set up to monitor the reading/recognition process on the basis of the microphone recordings and to determine a near vision acuity of the test person depending on the used font size/size, with a means being used for measuring the reading distance from the displayed text or displayed graphics (7) as chosen by the test person, **characterized in that** the computer device is set up to correct the near vision acuity to a distance-corrected value on the basis of a mathematical relationship by using the thus measured reading distance and the logarithm of the font size of a text which is still readable for the test person, with the means for measuring the reading distance being a stereophotogrammetric device with two video cameras (11, 11a), which is set up to determine the position of a point of reference in the face of the test person which can subsequently be placed in relation to the position of the presentation surface for calculating the reading distance.

2. An apparatus according to claim 1, **characterized by** an illumination system which is rigidly connected with the presentation area (3) and comprises at least one lighting means (5) and a light meter (6) for measuring the illumination prevailing in the region of the texts/graphics, and a control circuit for setting the luminous intensity originating from the at least one lighting means by way of the light meter (6) to a desired value of the illumination.

3. An apparatus according to claim 2, **characterized in that** the light meter (6) is a light sensor disposed in the presentation surface (3).

4. An apparatus according to one of the claims 1 to 3, **characterized in that** it comprises a luminance meter (6 + 6c).

5. An apparatus according to one of the claims 1 to 4, **characterized in that** it comprises a high-resolution display (3a), the luminance and/or luminance contrast and/or color or font or character size of which is measurable and adjustable in a computer-controlled way.

6. An apparatus according to claim 5, **characterized in that** a light meter (6d) is provided which rests on the display (3a) and which is arranged as a light sensor directed towards the display (3a).

7. An apparatus according to one of the claims 1 to 6, **characterized in that** the presentation surface can be inclined towards the test person about a pitch axis which is parallel to the right-left axis of the test person.

8. An apparatus according to one of the claims 1 to 7, **characterized in that** the computer device is additionally set up to derive the start and end of the reading process from the microphone recordings and to calculate a reading speed based on the length of the respectively read text.

9. An apparatus according to one of the claims 1 to 8, **characterized in that** the computer device or a software program executed therein is suitable for detecting, storing, statistically evaluating and printing the determined results.

10. An apparatus according to one of the claims 1 to 9, **characterized by** a device for mechanically/computer-controlled changing of the texts/graphics offered to the test person.

11. An apparatus according to claim 10, **characterized in that** the texts/graphics offered to the test person are shown on a screen.

12. An apparatus according to one of the claims 1 to 11, **characterized in that** the texts/graphics offered to the test person are realized in form of a reading board or reading strip.

**Revendications**

1. Dispositif destiné à déterminer la vision de près pendant la lecture/identification de textes/graphiques par un sujet testé, comportant une surface de présentation (3, 3a) sur laquelle peuvent être représentés un nombre de textes/graphiques (7) dans différentes tailles de caractères/grandeurs des graphiques, et un microphone (9) pour enregistrer les remarques du sujet testé pendant la lecture/identification des textes/graphiques, et comportant un ordinateur qui est configuré pour surveiller le processus de lecture/identification à l'appui des enregistrements par microphone et pour déterminer une vision de près du sujet testé en fonction de la taille des caractères/grandeur des graphiques utilisées, ledit dispositif utilisant un moyen destiné à mesurer la distance de lecture, choisie par le sujet testé, par rapport au texte représenté ou au graphique (7) représenté, **caractérisé en ce que** l'ordinateur est configuré pour corriger la vision de près à une valeur corrigée de distance, sur la base d'une relation mathématique, moyennant l'utilisation de la distance de lecture ainsi mesurée et du logarithme de la taille des caractères d'un texte pouvant encore être lu par le sujet testé, le moyen destiné à mesurer la distance de lecture étant un dispositif stéréophotogrammétrique avec deux caméras vidéo (11, 11a), lequel est configuré pour déterminer la position d'un point de référence sur le visage du sujet testé, laquelle peut ensuite être mise en relation avec la position de la surface de présentation pour le calcul de la distance de lecture.

2. Dispositif selon la revendication 1, **caractérisé par** un système d'éclairage, relié de manière fixe à la surface de présentation (3) et muni d'au moins un moyen d'éclairage (5) et d'un luminomètre (6) destiné à mesurer l'éclairage qui règne dans la zone des textes/graphiques, ainsi qu'un circuit de réglage pour le réglage, par l'intermédiaire du luminomètre (6), de l'intensité lumineuse, émise par ledit au moins un moyen d'éclairage, à une valeur d'éclairage souhaitée.

3. Dispositif selon la revendication 2, **caractérisé en ce que** le luminomètre (6) est un capteur de lumière situé dans la surface de présentation (3).

4. Dispositif selon l'une quelconque des revendications 1 à 3, **caractérisé en ce qu'**il comporte un luminancemètre (6 + 6c)

**5.** Dispositif selon l'une quelconque des revendications 1 à 4, **caractérisé en ce qu'**il comporte un écran (3a) à haute résolution, dont la luminance et/ou le contraste de luminance et/ou la couleur, voire la taille des lettres ou des caractères peuvent être mesurés, voire ajustés de manière commandée par ordinateur.

**6.** Dispositif selon la revendication 5, **caractérisé en ce qu'**il est prévu un luminomètre (6d) en appui contre l'écran (3a), lequel est réalisé sous la forme d'un capteur de lumière dirigé vers l'écran (3a).

**7.** Dispositif selon l'une quelconque des revendications 1 à 6, **caractérisé en ce que** la surface de présentation peut être inclinée vers le sujet testé autour d'un axe d'inclinaison parallèle à l'axe droite-gauche du sujet testé.

**8.** Dispositif selon l'une quelconque des revendications 1 à 7, **caractérisé en ce que** l'ordinateur est configuré en plus pour déduire à partir des enregistrements par microphone le début et la fin du processus de lecture et de calculer à partir de là une vitesse de lecture en fonction de la longueur du texte lu dans chaque cas.

**9.** Dispositif selon l'une quelconque des revendications 1 à 8, **caractérisé en ce que** l'ordinateur, plus précisément un programme informatique se déroulant sur celui-ci est apte à détecter, stocker, traiter statistiquement et imprimer les résultats calculés.

**10.** Dispositif selon l'une quelconque des revendications 1 à 9, **caractérisé par** un dispositif pour le changement mécanique/commandé par ordinateur des textes/graphiques proposés au sujet testé.

**11.** Dispositif selon la revendication 10, **caractérisé en ce que** les textes/graphiques proposés au sujet testé sont représentés sur un écran.

**12.** Dispositif selon l'une quelconque des revendications 1 à 11, **caractérisé en ce que** les textes/graphiques proposés au sujet testé sont réalisés en forme de tableaux de lecture ou sur une bande de lecture.

11

5

3

7

1

41 / 42

2

6b

12    6a    13    14

10

9    6

4

**Fig. 1**

11a

6d    3a

9

1a

10b

41 / 42

2a

12    10a    4a    6c    13a    14

**Fig. 1a**

Fig. 2

Fig. 2a

Fig. 3

Fig. 3a

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6422700 B
- US 6663241 B
- US 20060078858 A1
- US 5568209 A

**In der Beschreibung aufgeführte Nicht-Patentliteratur**

- **W. RADNER et al.** Eine neue Lesetafel zur gleichzeitigen Bestimmung von Lesevisus und Lesegeschwindigkeit. *Klin Monatsbl Augenheilkd,* 1998, vol. 213, 174-181
- **E. STIFTER et al.** Reliability of a standardized reading chart system: variance component analysis, test-retest and inter-chart reliability. *Graefe's Arch Clin Exp Ophthalmol,* 2004, vol. 242, 31-39